# EUROPEAN PATENT SPECIFICATION

(11) **EP 1 428 499 B1**
(45) Date of publication and mention of the grant of the patent: **18.10.2006**
(21) Application number: 03028205.7
(22) Date of filing: 09.12.2003
(51) Int. Cl.: A61K 8/37, A61K 8/42, A61K 8/46, A61K 8/73, A61K 8/891, A61K 8/895, A61Q 5/02, A61Q 5/12

(54) **Aqueous hair cleansing composition**
Wässrige Haarreinigungszusammensetzung
Composition aqueuse pour le nettoyage des cheveaux

(30) Priority: 10.12.2002 JP 2002357796
(43) Date of publication of application: 16.06.2004
(73) Proprietor: KAO CORPORATION, Tokyo 103-8210 (JP)
(72) Inventor: Tachizawa, Osamu, Kao Corporation Research Labor., Tokyo 131-8501 (JP); Terazaki, Hiroyuki, Kao Corporation Research Lab., Tokyo 131-8501 (JP)
(74) Representative: HOFFMANN EITLE

(56) References cited:
- EP-A- 0 190 010
- WO-A-97/35548
- US-A- 6 133 212
- US-A1- 2002 031 532

## Description

### FIELD OF THE INVENTION

The present invention relates to an aqueous hair cleansing composition containing sulfate surfactants, which exhibits benefits such as good foaming properties and a high lubricating foam quality during shampooing, provides smooth feeling during rinsing, imparts the hair with luster and manageability, has good low-temperature stability and is Substantially a non-irritant.

### BACKGROUND OF THE INVENTION

Alkyl sulfates typified by sodium dodecyl sulfate have most frequently been used as a cleansing component of an aqueous cleansing agent, because of their high detergency and its ability to generate a high foam volume. When an alkyl sulfate is used by itself, however, feel of the foam during shampooing is not satisfactory, because it causes friction between individual hairs. In order to improve the feel of the foam, polyoxyethylene-added alkyl sulfates having an ethylene oxide added between the alkyl group and the sulfuric acid group of an alkyl sulfate have been developed (alkyl ether sulfates). In particular, those added with 2 to 3 moles, on average, of ethylene oxide have become popular in shampoos because of its excellent feel upon cleansing. By the addition of ethylene oxide, however, the foaming speed is lowered greatly compared with that of alkyl sulfates. Mixed use of an alkyl sulfate and an alkyl ether sulfate improves foaming properties somewhat, but it cannot attain both the speedy formation of foam and good feel of the foam.

Alkyl ether sulfates added with 2.moles, on average, of ethylene oxide, which are typically and conventionally used are composed of about 20 wt.% of a 0 mole adduct, from 10 to less than 20 wt.% of 1, 2, and 3 mole adducts each, and the balance of 4 or greater mole adducts.

As a technique of improving the performance of a hair cleansing composition by adjusting the ratio of ethylene oxide added to an alkyl ether sulfate, disclosed is an aqueous shampoo composition (Japanese Patent No. 3260382) exhibiting high conditioning effects, which contains 5 to 50 wt.% of surfactant components including an alkyl ether sulfate added with 1 to 8 moles of ethylene oxide and an amphoteric surfactant, and less than 5 wt.% of an alkyl ether sulfate added with 1 mole or less of ethylene oxide. In this patent, it is not clear how the above-described 1 mole adduct is handled; nevertheless, this shampoo composition is believed to be insufficient in its foaming properties.

EP- 190010 and US-2002\031 532 disclose aqueous hair cleansing compositions comprising alkyl ether surfactants.

### SUMMARY OF THE INVENTION

The present invention provides an aqueous hair cleansing composition containing 5 to 30 wt.% of a sulfate surfactant which is composed of sulfates represented by the following formula (1):

R-O-(C₂H₄O)ₙ-SO₃M (1)

wherein, R represents a linear or branched C₈₋₁₈ alkyl or alkenyl group, n stands for 0 or a positive integer, and M represents sodium or ammonium, wherein 30 to 45 wt.% of the sulfates is a sulfate of formula (1) in which n=0, 18 to 27 wt.% of the sulfates is a sulfate of formula (1) in which n=1, 10 to 20 wt.% of the sulfates is a sulfate of formula (1) in which n=2, and the balance is a sulfate of formula (1) in which n is 3 or greater; and the total amount of the sulfates of formula (1) in which n is an integer of from 0. to 2 is 70 wt.% or greater based on all the sulfates.

### DETAILED DESCRIPTION OF THE INVENTION

The present invention relates to an aqueous hair cleansing composition which exhibits benefits which include good foaming properties and a high lubricating foam quality during shampooing, provides smooth feeling during rinsing, imparts the hair with luster and manageability, has good low-temperature stability and is substantially a non-irritant.

As a result of the investigation on the number of moles of ethylene oxide in polyoxyethylene-added alkyl sulfates; the present inventors have found that a hair cleansing composition satisfying the above-described demand is available by using a sulfate component containing various sulfates added with 0 to 2 moles of ethylene oxide each at a percentage within a predetermined range..

In the present invention, from the viewpoint of attaining both quick foaming and fine feel of foam, a sulfate surfactant having the number of moles of ethylene oxide addition falling within the above-described range is used. The sulfate component composed of 33 to 43 wt.% of a sulfate of formula (1) in which n=0, 20 to 25 wt.% of a sulfate of formula (1) in which n=1, 13 to 18 wt.% of a sulfate of formula (1) in which n=2 and the balance of sulfates of formula (1) in which n is 3 or greater is preferred, with that composed of 35 to 41 wt.% of a sulfate of formula (1) in which n=0, 21 to 23 wt.% of a sulfate of formula (1) in which n=1, 14 to 17 wt.% of a sulfate of formula (1) in which n=2 and the balance of sulfates of formula (1) in which n is 3 or greater is more preferred. From the same viewpoint, the percentage of the sulfates of formula (1) in which n is 0 to 2 in the sulfate surfactant is 70 wt.% or greater, with 70 to 85 wt.% being more preferred. Such a sulfate surfactant can be obtained by sulfating the alcohol ethoxylate, which has been obtained by adding 0.85 to 1.35 times the mole of ethylene oxide to a higher alcohol ROH, with 0.95 to 1.0 equivalent of SO₃ and then neutralizing the resulting sulfate with sodium hydroxide or ammonia.

The content of the sulfate surfactant in the aqueous hair cleansing composition of the present invention is 5 to 30 wt.%, preferably 7 to 23 wt.%, more preferably 10 to 20 wt.%.

To the aqueous hair cleansing composition of the present invention, a nonionic or amphoteric surfactant may be added as a surfactant other than the sulfate surfactant, in order to further improve the cleansing performance.

Examples of the nonionic surfactant include polyoxyalkylene sorbitan fatty acid esters, polyoxyalkylene sorbitol fatty acid esters, polyoxyalkylene glycerin fatty acid esters, polyoxyalkylene fatty acid esters, polyoxyalkylene alkyl ethers, polyoxyalkylene alkylphenyl ethers, polyoxyalkylene (hydrogenated) castor oils, sucrose fatty acid esters, polyglycerin alkyl ethers, polyglycerin fatty acid esters, fatty cid alkanolamides, and alkyl glycosides. Of these, alkyl glycosides, polyoxyalkylene (C₈ to C₂₂) fatty acid esters, polyoxyethylene sorbitan fatty acid esters, polyoxyethylene hydrogenated castor oils and fatty acid alkanolamides are preferred. As the fatty acid alkanolamides, those having a C₈₋₁₈, especially C₁₀₋₁₆ acyl group are preferred. As the fatty acid alkanolamides, either one of monoalkanolamides or dialkanolamides may be used, with those having a C₂₋₃ hydroxyalkyl group being preferred. Examples include oleic diethanolamide, palm kernel fatty acid diethanolamide, coconut oil fatty acid diethanolamide, lauric diethanolamide, polyoxyethylene coconut oil fatty acid monoethanolamide, coconut oil fatty acid monoethanolamide, lauric isopropanolamide and lauric monoethanolamide.

As the amphoteric surfactant, betaine surfactants can be used. Of these, preferred are alkyldimethylaminoacetic acid betaines and fatty acid amidopropylbetaines, of which fatty acid amidopropylbetaines is more preferred. As the fatty acid amidopropylbetaines, those having a C₈₋₁₈, especially C₁₀₋₁₆ acyl group are preferred, with laurylamidopropyl betaine, palm kernelamidopropyl betaine and cocamidopropyl betaine being more preferred.

In the aqueous hair cleansing composition of the present invention, the nonionic surfactant and amphoteric surfactant can be added as needed. Two or more of these surfactants may be used in combination. When the aqueous hair cleansing composition of the present invention is prepared in an aqueous liquid form, use of a fatty acid amidopropyl betaine or fatty acid alkanolamide is preferred, because it improves foaming power and imparts the composition with appropriate liquid properties.

The content of the nonionic surfactant in the aqueous hair cleansing composition of the present invention is preferably from 0 to 15 wt.%, more preferably from 0.5 to 10 wt.%, and even more preferably from 1 to 5 wt.%, while that of the amphoteric surfactant is preferably from 0 to 10 wt.%, more preferably from 0.5 to 8 wt.%, and even more preferably from 1 to 5 wt.%.

The aqueous hair cleansing composition of the present invention may contain a cationic polymer from the viewpoints of the texture and lubricity of foam, reduction in friction among individual hairs during cleansing and smoothness of hair during drying. Examples of the cationic polymer include cationic cellulose derivatives, cationic starches, cationic guar gum derivatives, homopolymers of a diallyl quaternary ammonium salt, diallyl quaternary ammonium salt/acrylamide copolymers, quaternized polyvinylpyrrolidone derivatives, polyglycol polyamine condensates, vinylimidazolium trichloride/vinylpyrrolidone copolymers, hydroxyethyl cellulose/dimethyldiallylammonium chloride copolymers, vinylpyrrolidone/quaternized dimethylaminoethyl methacrylate copolymers, polyvinylpyrrolidone/alkylaminoacrylate copolymers, polyvinylpyrrolidone/alkylaminoacrylate/vinyl caprolactum copolymers, vinylpyrrolidone/methacrylamidopropyl trimethylammonium chloride copolymers, alkylacrylamide/acrylate/alkylaminoalkylacrylamide/polyethyl ene glycol methacrylate copolymers, adipic acid/dimethylaminohydroxypropyl ethylene triamine copolymers ("Cartaretine", trade mark; product of Sandoz/USA), and cationic polymers described in Japanese Patent Application Laid-Open Nos. 139734/1978 and 36407/1985. Among them, cationic cellulose derivatives and cationic guar gum derivatives are preferred.

Two or more of these cationic polymers may be used in combination. The content thereof in the aqueous hair cleansing composition of the present invention is preferably from 0.02 to 5 wt.%, more preferably from 0.05 to 1 wt.%, and even more preferably from 0.1 to 0.3 wt.% in order to improve the foam quality upon cleansing, and manageability and touch of the hair after drying.

To the aqueous hair cleansing composition of the present invention, a conditioning component such as silicones may be added further for improving the finish of the hair after drying. For example, those described below can be used as the silicones.

### (1) Dimethylpolysiloxanes

Examples of dimethylpolysiloxanes include those represented by the following formula:

(Me)₃SiO-[(Me)₂SiO]_{d}-Si(Me)₃

wherein, Me represents a methyl group and d stands for a number of from 3 to 20000.

### (2) Amino-modified silicones

Various amino-modified silicones may be used. Particularly preferred are those having an average molecular weight of from about 3000 to 100000 and listed under the name of "Amodimethicone" in the third edition of CTFA Dictionary (Cosmetic Ingredient Dictionary/USA). These amino-modified silicones are preferably employed as an aqueous emulsion. Commercially available products include "SM 8704C" (product of Dow Corning Toray Silicone Co., Ltd.) and "DC 929" (product of Dow Corning Corporation).

### (3) Other silicones

Examples of the silicone other than those described above include polyether modified silicones, methylphenylpolysiloxanes, fatty acid modified silicones, alcohol modified silicones, alkoxy modified silicones, epoxy modified silicones, fluorine modified silicones, cyclic silicones and alkyl modified silicones.

Two or more of these silicones may be used in combination. The content thereof in the aqueous hair cleansing composition of the present invention is preferably from 0.01 to 10 wt.%, more preferably from 0.05 to 6 wt.%, even more preferably from 0.5 to 3 wt.%, and even more preferably from 2 to 3 wt.% from the viewpoints of the smoothness and improvement in luster of the hair after drying.

In addition, a pearling agent containing an ethylene glycol monoalkyl ester or ethylene glycol dialkyl ester may be incorporated in the cleansing composition to improve the texture and stability of the composition. Examples of the ethylene glycol monoalkyl ester include ethylene glycol monostearyl ester and ethylene glycol monobehenylester, while those of the ethylene glycol dialkyl ester include ethylene glycol distearyl ester and ethylene glycol dibehenyl ester. Two or more of them may be used in combination. The content of pearling agent in the aqueous hair cleansing composition of the present invention is preferably from 0.5 to 8 wt.%, more preferably from 1 to 5. wt.%, even more preferably from 2 to 3 wt.%. In view of improving stability of the cleansing composition, the weight ratio of the pearling agent to the sulfate surfactant (pearling agent/sulfate surfactant) in the hair cleansing composition of the present invention is preferably from 1/10 to 2/5, more preferably from 1/7 to 3/10, and even more preferably from 1/6 to 1/4.

The aqueous hair cleansing composition of the present invention has preferably a pH (after diluted to 20 times the weight with water, 25°C) of from 2 to 6, more preferably from 3 to 5, even more preferably from 3.5 to 4.5 when applied to the hair, in view of improving the luster and manageability of the hair. As the pH regulator, organic acids, especially α-hydroxy acids are preferred. Specifically, malic acid, citric acid, lactic acid and glycolic acid are preferred. Two or more of these organic acids may be used in combination. The content thereof in the aqueous hair cleansing composition of the present invention is preferably from 0.01 to 5 wt.%, more preferably from 0.1 to 3 wt.%, and even more preferably from 0.3 to 2 wt.% in view of improving the foam quality and flexibility of the hair upon cleansing. As another pH regulator, a base such as sodium hydroxide, potassium hydroxide or ammonium chloride may be used in combination with the organic acid.

The aqueous hair cleansing composition of the present invention may contain a viscosity regulator. Examples of the viscosity regulator include hydroxyethyl cellulose, methyl cellulose, polyethylene glycol, clay minerals, and salts (sodium chloride, ammonium chloride, sodium citrate and the like). Of these, salts, especially sodium chloride and sodium citrate are preferred. Two or more of the viscosity regulators may be used in combination. The content thereof in the aqueous hair cleansing composition of the present invention is preferably from 0.01 to 5 wt.%, more preferably from 0.05 to 3 wt.%, and even more preferably from 0.1 to 1.5 wt.% from the standpoints of the volume and quality of the foam.

The aqueous hair cleansing composition of the present invention may contain an agent for improving the finish of the hair such as benzyl alcohol, benzyloxy ethanol, panthenol, hydrolyzed silk protein, silk extract or the like to improve the touch or luster of the hair after drying. Two or more of the improving agents may be used in combination. The content thereof in the aqueous hair cleansing composition of the present invention is preferably from 0.01 to 5 wt.%, more preferably from 0.05 to 3 wt.%, and even more preferably from 0.1 to 1.5 wt.%.

The aqueous hair cleansing composition of the present invention may contain, in addition to the above-described components, components ordinarily used for hair cleansing compositions according to the intended use. Examples of such components include anti-dandruffs, vitamin preparations, bactericides, anti-inflammatory agents, antiseptics, chelating agents, humectants such as sorbitol and panthenol, colorants such as dyes and pigments, plant extracts, pearling agents such as titanium oxide, fragrances, coloring matters, ultraviolet absorbers, antioxidants and such components as described in ENCYCLOPEDIA OF SHAMPOO

### INGREDIENTS (MICELLE PRESS).

Although the hair cleansing composition of the present invention can be provided in either a liquid form or a gel form as needed, the liquid form using a lower alcohol or water as a solvent, especially a liquid form using water is preferred.

The following examples further describe and demonstrate embodiments of the present invention. The examples are given solely for the purpose of illustration and are not to be construed as limitations of the present invention.

### -Examples-

### Preparation Example 1

In a pressure-resistant and air tight reaction apparatus were charged 2000 g of "Kalcol 2470" (trade name; product of Kao Corp., dodecyl alcohol: tetradecyl alcohol = about 3:1) and 1.45 g of potassium hydroxide, followed by dehydration at 110°C and 10 mmHg for 30 minutes. The temperature in the system was then raised to 165°C. Into the apparatus, 456 g of ethylene oxide was pressed and addition reaction was performed for 30 minutes without changing the temperature. The reaction mixture was then cooled to 80°C, and neutralized with 1.3 g of acetic acid, whereby an ethylene oxide adduct of the raw material alcohol was obtained.

A sulfating reaction was conducted using 1793 g of the resulting mixture and 607 g of a sulfuric acid gas at 40°C. After the reaction, the product thus obtained was neutralized with 132 g of a 23 wt.% aqueous solution of sodium hydroxide and 556 g of deionized water. The concentration and pH of the product were regulated by using a 23 wt.% aqueous solution of sodium hydroxide, 75 wt.% phosphoric acid and deionized water, whereby 10000 g of a 25 wt.% aqueous solution of Sulfate 1 as listed in Table 1 was obtained.

In accordance with the Japanese Standards of Cosmetic Ingredients[published in 1999], the sulfate thus obtained was analyzed with regard to the sodium salt, sulfate, anion and EO chain.

### Preparation Example 2

In a similar manner to Preparation Example 1 except that the reaction ratio of the raw materials was changed, a 25 wt.% aqueous solution of Sulfate 2 as listed in Table 1 was obtained.

In accordance with the Japanese Standards of Cosmetic Ingredients[published in 1999], the sulfate thus obtained was analyzed with regard to the sodium salt, sulfate, anion and EO chain.

### Preparation Example 3

A sulfating reaction was performed at 40°C by using 1793 g of the ethylene oxide adduct of the raw material alcohol obtained in Preparation Example 1 and 607 g of a sulfuric acid gas. After the reaction, the product was neutralized with 150 g of 28 wt.% aqueous ammonia and 600 g of deionized water. By using 28 wt.% aqueous ammonia and deionized water, the concentration and pH of the product were regulated, whereby 10000 g of a 25 wt.% aqueous solution of Sulfate 3 as listed in Table 1 was obtained.

In accordance with the Japanese Standards of Cosmetic Ingredients[published in 1999], the sulfate thus obtained was analyzed with regard to the sulfate, anion and EO chain. Examples 1 to 11 and Comparative Examples 1 to 4

By using the sulfate surfactants (sodium lauryl ether sulfates) as listed in Table 1, hair cleansing compositions as shown in Tables 2 and 3 were prepared and their foaming speed, lubricity upon shampooing, luster and manageability of the hair after drying were evaluated. The composition ratio in Table 1 was measured by gas chromatography. (Foaming speed)

By using 1.5 mL of a sample to be evaluated and 0.3 mL of a model sebum, the foam volume was measured under the conditions and using the apparatus as described in paragraphs [0053] and [0054] in Japanese Patent Application Laid-Open No. 73584/1998 and the foaming speed was evaluated based on the time until the foam volume reached 25 mL. Criteria for evaluation
- A:: less than 100 seconds
- B:: not less than 100 seconds but less than 200 seconds
- C:: not less than 200 seconds but less than 300 seconds
- D:: not less than 300 seconds

### (Lubricity of the hair during shampooing)

After a human hair bundle of 25 cm in length, 5.5 cm in width and 10 g in weight was rinsed lightly with warm water at 40°C and excess water was removed, 0.5 g of the hair cleansing composition was applied thereto and foamed sufficiently for about 30 seconds. Then the lubricity of the hair bundle with foams was subjected to organoleptic evaluation by a panel of 5 experts. The composition was evaluated by the sum of their scores.

### Criteria for evaluation:

- 4:: very lubricious
- 3:: Somewhat lubricious
- 2:: Not so lubricious
- 1:: Not lubricious

### (Luster and manageability of the hair after drying)

The hair bundle treated similarly as in the lubricity evaluation was rinsed for 30 seconds with running water (2L/min) at 40°C. The hair bundle was towel-dried sufficiently and then dried naturally. After drying, the luster and manageability of the hair was visually evaluated by a panel of 5 experts. The composition was evaluated by the sum of their scores.

### Criteria for evaluation

- 4:: Good
- 3:: Somewhat good
- 2:: Not so good
- 1:: Not good

**Table 1**

| Composition ratio (wt.%) of sulfate surfactant components with various numbers of moles of EO added | | | | | | | | | | | | | | |
|---|---|---|---|---|---|---|---|---|---|---|---|---|---|---|
| | n in formula (1) | | | | | | | | | | | | | |
| | 0 | 1 | 2 | 3 | 4 | 5 | 6 | 7 | 8 | 9 | 10 | 11 | 12 | 13 |
| Sulfate 1 | 40.64 | 22.29 | 14.80 | 8.68 | 4.90 | 2.99 | 1.91 | 1.32 | 0.92 | 0.62 | 0.42 | 0.27 | 0.17 | 0.08 |
| Sulfate 2 | 34.29 | 21.41 | 16.59 | 10.09 | 5.77 | 3.60 | 2.35 | 1.72 | 1.29 | 0.96 | 0.71 | 0.49 | 0.43 | 0.31 |
| Sulfate 3 | 40.64 | 22.29 | 14.80 | 8.68 | 4.90 | 2.99 | 1.91 | 1.32 | 0.92 | 0.62 | 0.42 | 0.27 | 0.17 | 0.08 |
| Comparative Sulfate 1 | 46.43 | 10.70 | 10.73 | 8.83 | 6.45 | 4.71 | 3.43 | 2.65 | 2.04 | 1.56 | 1.19 | 0.82 | 0.41 | 0.04 |
| Comparative Sulfate 2 | 46.01 | 11.18 | 12.28 | 11.34 | 7.96 | 5.58 | 4.16 | 1.50 | 0.00 | 0.00 | 0.00 | 0.00 | 0.00 | 0.00 |
| Comparative Sulfate 3 | 19.97 | 15.99 | 16.03 | 13.20 | 9.64 | 7.04 | 5.13 | 3.96 | 3.05 | 2.33 | 1.78 | 1.23 | 0.62 | 0.05 |

| | | | | | | | | | | | | | | |
|---|---|---|---|---|---|---|---|---|---|---|---|---|---|---|
| Sulfate 1: obtained In Preparation Examples 1 Sulfate 2: obtained In Preparation Example 2 Sulfate 3: obtained in Preparation Example 3 Comparative Sulfates 1 and 2: mixture of a lauryl ether sulfate added with 2.0 moles, on average, of EO (Comparative Sulfate 3) and a lauryl sulfate Comparative Sulfate 3: lauryl ether sulfates added with 2.0 moles, on average, of EO ("Emal 227-PH11", trade name; product of Kao Corp.) | | | | | | | | | | | | | | |

**Table 2**

| Component (wt.%) | Examples | | | | | | | | Comparative Examples | | | |
|---|---|---|---|---|---|---|---|---|---|---|---|---|
| | 1 | 2 | 3 | 4 | 5 | 6 | 7 | 8 | 1 | 2 | 3 | 4 |
| Sulfate 1 | 10 | 15 | | | 10 | 12 | | | | | | |
| Sulfate 2 | | | 10 | 8 | | | | | | | | |
| Sulfate 3 | | | | | | | 12 | 15 | | | | |
| Comparative Sulfate 1 | | | | | | | | | 15 | | | |
| Comparative Sulfate 2 | | | | | | | | | | 10 | | |
| Comparative Sulfate 3 | | | | | | | | | | | 15 | 12 |
| Laurylamidopropyl betaine | | | | 2 | | | | | | | 2 | |
| Myristyl alcohol | 1 | 1 | 1 | | 1 | 1 | 1 | 1 | 1 | 1 | | 1 |
| Ethyelne glycol distearate | | | 2 | 2 | 2 | 3 | 3 | 3 | | 2 | 2 | 3 |
| Distearyl ether | | 2 | | | | | | | 2 | | | |
| Behenyl alcohol | 2 | | 2 | | 2 | | | | | 2 | | |
| Cationic hydroxyethyl cellulose | 0.5 | | 0.3 | | 0.3 | 0.2 | 0.2 | 0.3 | | 0.3 | | 0.2 |
| Cationic guar gum | | 0.5 | 0.2 | 0.5 | 0.2 | 0.3 | 0.3 | | 0.5 | 0.2 | 0.5 | 0.3 |
| Amino-modified silicone | | | | | | 0.1 | 0.1 | | | | | 0.1 |
| Dimethicone (gum viscosity: 8 million mm2/s, average particle size; 0.5µm) | | | | | | 1.2 | 1.2 | 0.5 | | | | 1.2 |
| Malic acid | 0.75 | 0.75 | 0.75 | 0.75 | 0.03 | 0.75 | 0.75 | 0.75 | 0.75 | 0.03 | 0.75 | 0.75 |
| Sodium chloride | | | | | | 0.2 | | | | | | 0.2 |
| Purified water | Balance | Balance | Balance | Balance | Balance | Balance | Balance | Balance | Balance | Balance | Balance | Balance |
| pH (when diluted to 20 times the weight with water, 25°C) | 3.7 | 3.7 | 3.7 | 3.7 | 5.5 | 3.7 | 3.7 | 3.7 | 3.7 | 5.5 | 3.7 | 3.7 |
| Foaming speed | A | A | A | A | A | A | A | A | B | B | D | D |
| Lubricity of foam | 18 | 18 | 20 | 18 | 19 | 20 | 20 | 19 | 15 | 9 | 7 | 10 |
| Luster and manageability | 19 | 20 | 20 | 18 | 15 | 20 | 20 | 20 | 18 | 6 | 18 | 18 |

**Table 3**

| Component (wt.%) | Examples | | |
|---|---|---|---|
| | 9 | 10 | 11 |
| Sulfate 1 | | | 13 |
| Sulfate 3 | 12 | 12 | |
| Myristyl alcohol | 0.5 | 0.3 | 1 |
| Ethylene glycol distearate | 2.4 | 3 | 2.5 |
| Benzyl alcohol | 0.5 | 0.3 | |
| Panthenol | | 0.1 | |
| Silk extract | | | 0.1 |
| Cationic hydroxyethyl cellulose | 0.3 | 0.1 | |
| Cationic guar gum | 0.2 | 0.3 | 0.5 |
| Amino-modified silicone | 0.1 | | 0.2 |
| Dimethicone (gum viscosity: 8 million mm²/s, average particle size: 0.5 µm) | 1 | 1.5 | 1.2 |
| Malic acid | 0.2 | | 0.5 |
| Lactic acid | 0.15 | 0.3 | |
| Citric acid | 1 | | |
| Glycolic acid | | 0.1 | |
| Sodium chloride | | 0.2 | |
| Sodium citrate | 1 | | |
| Ammonium chloride | | | 0.3 |
| Purified water | Balance | Balance | Balance |
| pH (when diluted to 20 times the weight with water, 25°C) | 3.7 | 3.7 | 3.7 |
| Foaming speed | A | A | A |
| Lubricity of foam | 20 | 20 | 19 |
| Luster and manageability | 20 | 19 | 18 |

### Example 12 Pearlescent shampoo

| | (wt.%) |
|---|---|
| Sulfate 1 | 15.0 |
| Laurylamidopropyl betaine | 0.5 |
| Cocoyl monoethanolamide | 0.3 |
| Ethylene glycol distearate | 2.0 |
| Cationic hydroxyethyl cellulose | 0.2 |
| Glycerin | 1.0 |
| Salicylic acid | Amount to adjust pH to 3.7 |
| Deionized water | Balance |

The resulting shampoo exhibited quick foaming and good lubricity of foam and the luster and manageability of the hair shampooed therewith was good.

### Example 13: Conditioning shampoo

| | (wt.%) |
|---|---|
| Sulfate 1 | 13.0 |
| Cocoyl monoethanolamide | 0.7 |
| Myristyl alcohol | 1.0 |
| Distearyl ether | 3.0 |
| Cationic hydroxyethyl cellulose | 0.5 |
| Glycerin | 1.0 |
| Sodium chloride | 1.0 |
| Lactic acid | 0.5 |
| Malic acid | Amount to adjust pH to 3.7 |
| Deionized water | Balance |

The resulting shampoo exhibited quick foaming and good lubricity of foam and the luster and manageability of the hair shampooed therewith was good.

### Example 14: Conditioning shampoo

| | (wt.%) |
|---|---|
| Sulfate 1 | 13.0 |
| Cocoyl monoethanolamide | 1.0 |
| Myristyl alcohol | 1.0 |
| Cetanol | 0.5 |
| Ethylene glycol distearate | 3.0 |
| Cationic hydroxyethyl cellulose | 0.1 |
| Cationic guar gum | 0.3 |
| Glycerin | 1.0 |
| Dimethicone (400000 mPa·s) | 0.5 |
| Sodium chloride | 0.2 |
| Benzyl alcohol | 0.5 |
| Malic acid | 0.7 |
| Deionized water | Balance |

The resulting shampoo exhibited quick foaming and good lubricity of foam and the luster and manageability of the hair shampooed therewith was good.

### Example 15: Conditioning shampoo

| | (wt.%) |
|---|---|
| Sulfate 1 | 10.0 |
| Laurylamidopropylbetaine | 3.0 |
| Myristyl alcohol | 1.0 |
| Cetanol | 0.5 |
| Behenyltrimonium chloride | 0.5 |
| Ethylene glycol distearate | 2.0 |
| Cationic guar gum | 0.3 |
| Panthenol | 0.1 |
| Hydrolyzed silk protein | 0.3 |
| Sodium chloride | 1.0 |
| Malic acid | 0.75 |
| Lactic acid | 0.75 |
| Sodium hydroxide | Amount to adjust pH to 3.5 |
| Deionized water | Balance |

The resulting shampoo exhibited quick foaming and good lubricity of foam and the luster and manageability of the hair shampooed therewith was good.

### Example 16: Pearlescent anti-dandruff shampoo

| | (wt.%) |
|---|---|
| Sulfate 1 | 13.0 |
| Cocoyl monoethanolamide | 0.5 |
| Myristyl alcohol | 1.0 |
| Cetanol | 0.5 |
| Distearyl ether | 2.0 |
| Cocoylbenzalconium chloride | 0.5 |
| Cationic hydroxyethyl cellulose | 0.3 |
| Cationic guar gum | 0.3 |
| Glycerin | 1.0 |
| Dimethicone (400000 mPa·s) | 1.0 |
| Sodium hydroxide | 0.2 |
| Benzyloxy ethanol | 0.5 |
| Malic acid | 0.7 |
| Deionized water | Balance |

The resulting shampoo exhibited quick foaming and good lubricity of foam and the luster and manageability of the hair shampooed therewith was good.

## Claims

1. An aqueous hair cleansing composition comprising 5 to 30 wt.% of a sulfate surfactant which is composed of sulfates represented by the following formula (1):
R-O-(C₂H₄O)ₙ-SO₃M (1)
wherein, R represents a linear or branched C₈₋₁₈ alkyl or ' alkenyl group, n stands for 0 or a positive integer, and M represents sodium or ammonium, wherein 30 to 45 wt.% of the sulfates is a sulfate of formula (1) in which n=0, 18 to 27 wt.% of the sulfates is a sulfate of formula (1) in which n=1, 10 to 20 wt.% of the sulfates is a sulfate of formula (1) in which n=2, and the balance is a sulfate of formula (1) in which n is 3 or greater; and the total amount of the sulfates of formula (1) in which n is an integer of from 0 to 2 is 70 wt.% or greater based on all the sulfates.

2. The aqueous hair cleansing composition of Claim 1, further comprising a nonionic or amphoteric surfactant.

3. The aqueous hair cleansing composition of Claim 1 or 2, further comprising a cationic polymer.

4. The aqueous hair cleansing composition of any one of Claims 1 to 3, further comprising a silicone.

5. The aqueous hair cleansing composition of any one of Claims 1 to 4, further comprising a pearling agent containing either an ethylene glycol monoalkyl ester or an ethylene glycol dialkyl ester.

## Patentansprüche

1. Wässrige Haarreinigungszusammensetzung, umfassend 5 bis 30 Gew.% eines Sulfattensides, das sich aus Sulfaten mit der folgenden Formel (1) zusammensetzt:
R-O-(C₂H₄O)ₙ-SO₃M (1)
worin R eine lineare oder verzweigte C₈₋₁₈-Alkyl- oder -Alkenylgruppe ist, n für 0 oder eine positive ganze Zahl steht und M Natrium oder Ammonium-ist, worin 30 bis 45 Gew.% der Sulfate ein Sulfat mit der Formel (1) sind, worin n=0, 18 bis 27 Gew.% der Sulfate ein Sulfat der Formel (1) sind, worin n=1 ist, 10 bis 20 Gew.% der Sulfate ein Sulfat der Formel (1) sind, worin n=2 und der Rest ein Sulfat der Formel (1) ist, worin n 3 oder mehr ist, und die Gesamtmenge der Sulfate der Formel (1), worin n eine ganze Zahl von 0 bis 2 ist, 70 Gew.-% oder mehr ist, bezogen auf alle Sulfate.

2. Wässrige Haarreinigungszusammensetzung nach Anspruch 1, weiterhin umfassend ein nichtionisches oder amphoteres Tensid.

3. Wässrige Haarreinigungszusammensetzung nach Anspruch 1 oder 2, weiterhin umfassend ein kationisches Polymer.

4. Wässrige Haarreinigungszusammensetzung nach einem der Ansprüche 1 bis 3, weiterhin umfassend ein Silikon.

5. Wässrige Haarreinigungszusammensetzung nach einem der Ansprüche 1 bis 4, weiterhin umfassend ein Perlmittel mit einem Ethylenglycolmonoalkylester oder einem Ethylenglycoldialkylester.

## Revendications

1. Composition aqueuse de nettoyage capillaire comprenant 5 à 30 % en pds d'un tensioactif de type sulfate qui est composé des sulfates représentés par la formule suivante (1) :
R-O-(C₂H₄O)ₙ-SO₃M (1)
dans laquelle, R représente un groupe alkyle ou alcényle en C₈₋₁₈ linéaire ou ramifié, n représente 0 ou un nombre entier positif, et M représente le sodium ou l'ammonium, dans laquelle 30 à 45 % en pds des sulfates est un sulfate de formule (1) dans laquelle n = 0, 18 à 27 % en pds des sulfates est un sulfate de formule (1) dans laquelle n = 1, 10 à 20 % en pds des sulfates est un sulfate de formule (1) dans laquelle n = 2, et le reste est un sulfate de formule (1) dans laquelle n prend la valeur de 3 ou plus ; et la quantité totale des sulfates de formule (1) dans laquelle n représente un nombre entier de 0 à 2 est de 70 % en pds ou plus basée sur tous les sulfates.

2. Composition aqueuse de nettoyage capillaire selon la revendication 1, comprenant en outre un tensioactif non ionique ou amphotère.

3. Composition aqueuse de nettoyage capillaire selon la revendication 1 ou 2, comprenant en outre un polymère cationique.

4. Composition aqueuse de nettoyage capillaire selon l'une quelconque des revendications 1 à 3, comprenant en outre une silicone.

5. Composition aqueuse de nettoyage capillaire selon l'une quelconque des revendications 1 à 4, comprenant en outre un agent nacrant contenant soit un ester monoalkylique de l'éthylène glycol soit un ester dialkylique de l'éthylène glycol.
